# EUROPEAN PATENT APPLICATION

(11) **EP 4 663 213 A1**
(43) Date of publication of application: **17.12.2025**
(21) Application number: 24753172.6
(22) Date of filing: 29.01.2024
(51) Int. Cl.: A61M 1/00, A61M 39/20

(54) **CAP FOR MEDICAL INSTRUMENT**

(30) Priority: 09.02.2023 JP 2023018152
(71) Applicant: Tsukada Medical Research Co., Ltd., Tokyo 161-0034 (JP)
(72) Inventor: KOHDA, Isamu, Tokyo 161-0034 (JP); KOBAYASHI, Teruyuki, Tokyo 161-0034 (JP)
(74) Representative: Grünecker Patent- und Rechtsanwälte PartG mbB
(86) International application number: PCT/JP2024/002637
(87) International publication number: WO 2024/166728

(57) **Abstract**

An object of the present invention is to provide a medical instrument cap of which lid closing force is adjustable. A medical instrument cap (100) includes a connection plug main body (10) that has attached thereto a main body-side magnetic member (15) and that has formed therein a flow path (14) penetrating from a base end portion (12) formed to be connectable to an external member to an opening portion (13) positioned on an opposite side, and a lid portion (30) that has attached thereto a lid-side magnetic member (32) generating attractive force to the main body-side magnetic member (15) and that is configured to close the flow path (14) by abutting against the opening portion (13) of the connection plug main body (10). At least one of the main body-side magnetic member (15) and the lid-side magnetic member (32) is configured so that magnitude of the attractive force acting between the main body-side magnetic member (15) and the lid-side magnetic member (32) is adjustable.

## Description

### TECHNICAL FIELD

The present invention relates to a medical instrument cap that functions as an external open/close valve for a medical instrument placed in a human body, such as a urethral catheter.

### BACKGROUND ART

Conventionally, as for urethral catheters for the purpose of managing urine disposal, users themselves are supposed to place a urethral catheter and to dispose of urine. Thus, there is a demand for a urethral catheter that can easily be operated even by users who find it difficult to operate urethral catheters due to hand dexterity problems, such as patients with a cerebrovascular disorder, a spinal cord injury, or the like. To solve this problem, a medical instrument cap has been proposed (see Patent Literature 1, for example) which functions as an external open/close valve for a urethral catheter and provides assistance with magnetic attractive force at the time of closing a lid.

### CITATION LIST

### PATENT LITERATURE

PTL 1: Japanese Patent Laid-Open No. 9-206370

### SUMMARY OF INVENTION

### TECHNICAL PROBLEM

However, when being shipped from a factory, the conventional medical instrument cap described above has incorporated in advance the magnet to be used for closing the lid. Thus, regardless of degrees of hand dexterity of the users, the incorporated magnet has the same strength. For this reason, there is a demand, particularly from elderly users and the like who have weaker gripping or pinching force, for a medical instrument cap that has lower magnetic attractive force than that of the conventional medical instrument cap. However, for younger users who do strenuous exercise such as basketball players on wheelchairs, for example, a medical instrument cap having lower magnetic attractive force poses a risk of the lid being opened by shock and possible urine leakage.

It is an object of the present invention to solve the problems of the conventional technique described above and to provide a medical instrument cap of which the force used to close the lid is adjustable.

### SOLUTION TO PROBLEM

The present invention provides a medical instrument cap, including a connection plug main body that has attached thereto a main body-side magnetic member and that has formed therein a flow path penetrating from a base end portion formed to be connectable to an external member to an opening portion positioned on an opposite side, and a lid portion that has attached thereto a lid-side magnetic member generating attractive force to the main body-side magnetic member and that is configured to close the flow path by abutting against the opening portion of the connection plug main body. At least one of the main body-side magnetic member and the lid-side magnetic member is configured so that magnitude of the attractive force acting between the main body-side magnetic member and the lid-side magnetic member is adjustable.

In this case, the opening portion may be formed to have a circular cylindrical shape and may have a screw thread formed on an outer circumference thereof. The main body-side magnetic member may be attached to a base portion that is formed to have an annular shape and that has a screw thread formed on an inner circumference thereof, so as to be screwed together with the opening portion via the base portion. The base portion may include a circular cylindrical portion to be screwed together with the opening portion. The base portion may have an anti-slip formed along an outer circumference thereof. The connection plug main body may have formed therein clearances through which an outer circumference of the base portion is exposed from a pair of locations positioned opposite to each other while being centered on an axial center of the opening portion. At least one of the main body-side magnetic member and the lid-side magnetic member may removably be attached to the connection plug main body or the lid portion.

### ADVANTAGEOUS EFFECTS OF INVENTION

The present invention can provide a medical instrument cap of which the force used to close the lid is adjustable.

### BRIEF DESCRIPTION OF DRAWINGS

[Fig. 1] Fig. 1 is a perspective view of a medical instrument cap in a state where a lid portion is open.
[Fig. 2] Fig. 2 is an exploded perspective view of the medical instrument cap.
[Fig. 3] Fig. 3 is a schematic cross-sectional view of the medical instrument cap in a state where an action of a magnet is increased.
[Fig. 4] Fig. 4 is a schematic cross-sectional view of the medical instrument cap in a state where the action of the magnet is decreased.

### DESCRIPTION OF EMBODIMENTS

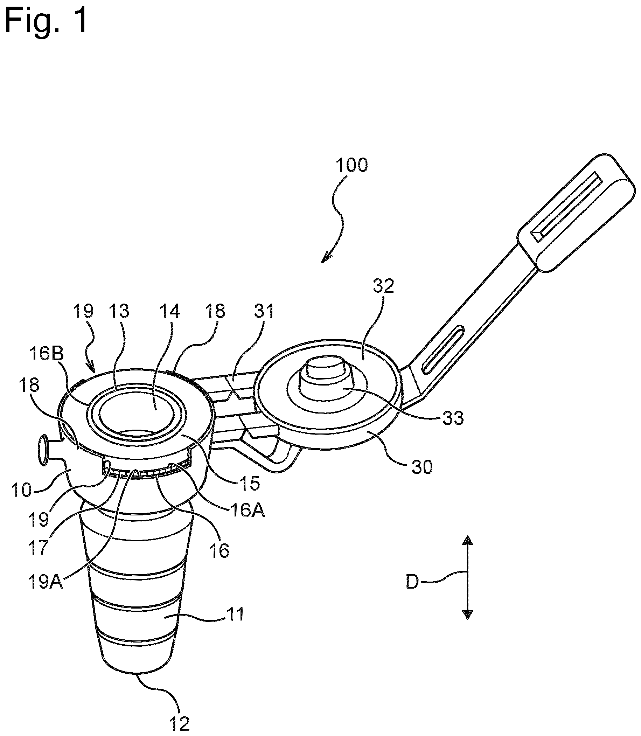

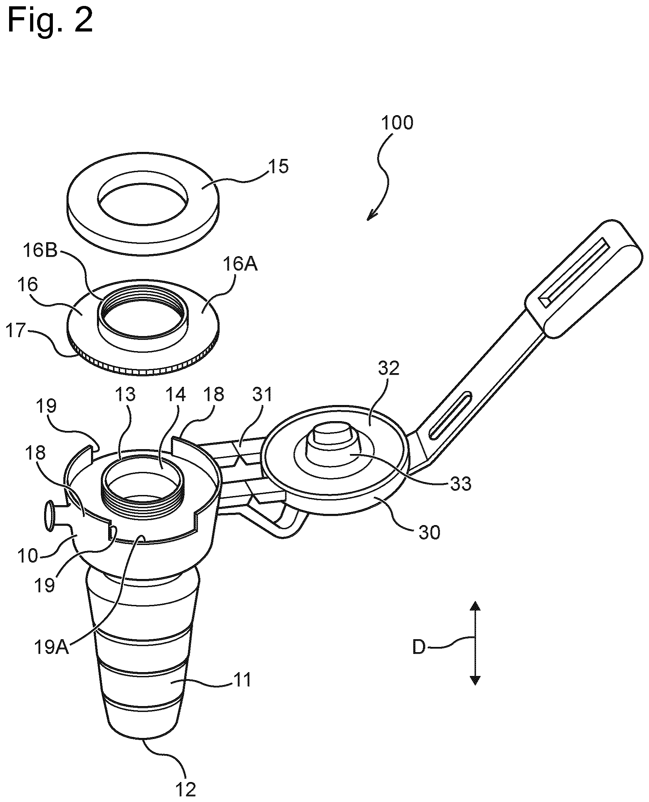

The following will describe a medical instrument cap according to an embodiment of the present invention, with reference to the drawings. Fig. 1 is a perspective view of the medical instrument cap in a state where a lid portion is open. Fig. 2 is an exploded perspective view of the medical instrument cap.

A medical instrument cap 100 is provided so as to be connected to a urethral catheter, a urine bag, or the like, via a urine disposal tube (not shown). The medical instrument cap 100 is formed by using resin having elasticity and includes, as shown in Fig. 1, a connection plug main body 10 and a lid portion 30 that are integrally formed.

The connection plug main body 10 has formed therewith a connection portion 11 that is connectable to an external member such as a urethral catheter, a urine bag, or the like, via a tube or the like. The connection plug main body 10 has formed therein a flow path 14 penetrating from a base end portion 12 formed to be fluidly connected to the external member, to an opening portion 13 positioned on the opposite side. The flow path 14 is formed to have a circular shape so as to penetrate through the centers of the connection portion 11 and the opening portion 13. The opening portion 13 is formed to have a circular cylindrical shape and has, on the outer circumference of the circular cylindrical shape, a screw thread formed so as to spirally extend along the extending direction of the circular cylindrical shape.

The connection plug main body 10 has attached thereto a magnet (a main body-side magnetic member) 15 formed to have an annular shape. The magnet 15 is attached to the opening portion 13 via a base portion 16.

As shown in Fig. 2, the base portion 16 includes a circular cylindrical portion 16B protruding, in a circular cylindrical shape, along the inner circumference of an annular portion 16A formed to have an annular shape. The magnet 15 is fitted with the circular cylindrical portion 16B and is adhered onto the base portion 16. Formed on the inner circumference of the annular portion 16A and the inner circumference of the circular cylindrical portion 16B is a spiral screw thread extending from the annular portion 16A and continued onto the circular cylindrical portion 16B. The inner circumference of the base portion 16 is configured to be screwed together with the outer circumference of the opening portion 13. In other words, the magnet 15 of the connection plug main body 10 is screwed together with the opening portion 13 via the base portion 16. With this arrangement, as a result of a user rotating the base portion 16, the base portion 16 is configured to move in the directions of the arrow D in the drawing, along the opening portion 13 formed to have the circular cylindrical shape.

The base portion 16 has an anti-slip 17 formed along the outer circumference of the annular portion 16A, for the purpose of preventing fingers of the user from slipping on the base portion 16 during operations by the user. In the anti-slip 17, notches each extending along the extending direction of the circular cylindrical portion 16B are arranged over the entire circumference of the annular portion 16A.

The connection plug main body 10 is formed so that a lateral wall 18 having a circular cylindrical shape covers the outer circumference of the base portion 16. On the lateral wall 18, a hinge portion 31 for opening and closing the lid portion 30 is integrally formed therewith. The lateral wall 18 has clearances 19 formed in a pair of locations that are positioned perpendicular to the direction in which the lid portion 30 opens from the connection plug main body 10 and are positioned opposite to each other while being centered on the axial center of the opening portion 13. With this configuration, the anti-slip 17 of the base portion 16, together with the outer circumferential surface of the magnet 15, is exposed to the outside through the pair of clearances 19, 19. Here, the anti-slip 17 of the base portion 16 protrudes farther outwardly in the radial direction, relative to the outer circumferential surface of the magnet 15. The anti-slip 17 is positioned between the magnet 15 and edge portions 19A of the clearances 19, 19 which are arc-shaped and formed on the connection portion 11 side. Accordingly, during normal use, i.e., while the user is not operating the base portion 16, the anti-slip 17 is not easily touched by the hands of the user or the like.

The lid portion 30 has formed therewith a shaft portion raised from a central portion of the inner surface. The shaft portion has incorporated therewith a seal portion 33 having a circular cylindrical shape and structured by using silicone rubber, for example. The lid portion 30 is provided integrally with the connection plug main body 10, so as to be able to swing on the hinge portion 31. When being closed while the hinge portion 31 is used as a fulcrum, the lid portion 30 is configured to close the flow path 14 by abutting against the opening portion 13 of the connection plug main body 10.

The lid portion 30 has incorporated therein an iron plate (a lid-side magnetic member) 32 provided in a position facing the magnet 15 of the connection plug main body 10 while the flow path 14 is in a closed state, so as to generate attractive force between the iron plate 32 and the magnet 15 of the connection plug main body 10. Because the magnet 15 of the connection plug main body 10 is attached onto the base portion 16, the medical instrument cap 100 according to the present embodiment is configured to be able to change the distance between the iron plate 32 of the lid portion 30 in a closed state and the magnet 15. Consequently, by moving the base portion 16, it is possible to adjust magnitude of the attractive force generated between the magnet 15 of the connection plug main body 10 and the iron plate 32 of the lid portion 30.

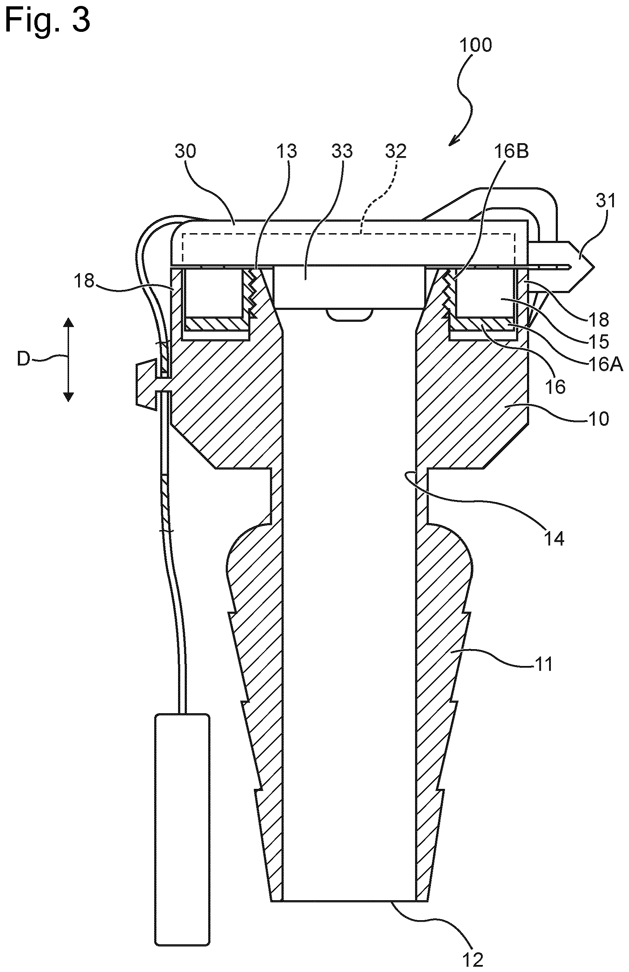

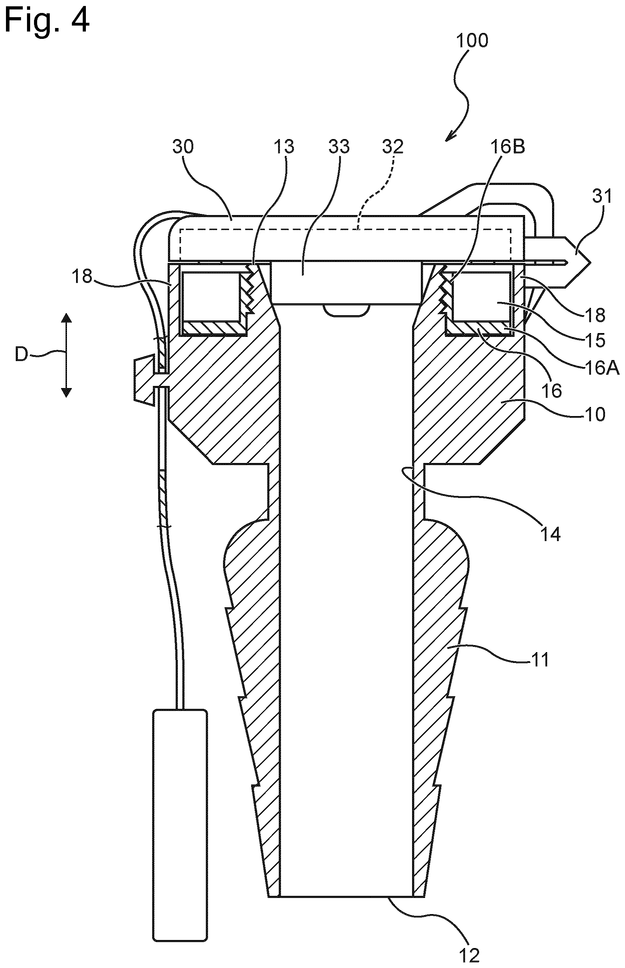

Next, the adjustment of the attractive force acting between the connection plug main body 10 and the lid portion 30 will be explained. Fig. 3 is a schematic cross-sectional view of the medical instrument cap in a state where the action of the magnet is increased. Fig. 4 is a schematic cross-sectional view of the medical instrument cap in a state where the action of the magnet is decreased.

When the magnetic force acting between the connection plug main body 10 and the lid portion 30 is to be increased, the base portion 16 together with the magnet 15 is moved closer to the iron plate 32 of the lid portion 30, as shown in Fig. 3. When bringing the base portion 16 closer, together with the magnet 15, to the iron plate 32 of the lid portion 30, the user presses their fingers against the anti-slip 17 of the base portion 16 through the clearances 19, 19 provided on both sides and rotates the base portion 16 . In that situation, when rotating the base portion 16 counterclockwise as seen from the lid portion 30 side, the base portion 16 moves toward the iron plate 32 of the lid portion 30 along the opening portion 13, so that the magnetic force acting between the connection plug main body 10 and the lid portion 30 increases.

On the contrary, when the magnetic force acting between the connection plug main body 10 and the lid portion 30 is to be decreased, the base portion 16 together with the magnet 15 is moved away from the iron plate 32 of the lid portion 30, as shown in Fig. 4. When bringing the base portion 16 closer, together with the magnet 15, to the iron plate 32 of the lid portion 30, the user presses their fingers against the anti-slip 17 of the base portion 16 through the clearances 19, 19 provided on both sides and rotates the base portion 16. In that situation, when rotating the base portion 16 clockwise as seen from the lid portion 30 side, the base portion 16 moves away from the iron plate 32 of the lid portion 30 along the opening portion 13, so that the magnetic force acting between the connection plug main body 10 and the lid portion 30 decreases.

The medical instrument cap 100 according to the present embodiment includes the connection plug main body 10 that has formed therein the flow path 14 penetrating from the base end portion 12 formed to be connectable to an external member to the opening portion 13 positioned on the opposite side and that has the magnet 15 attached thereto, and the lid portion 30 that has attached thereto the iron plate 32 for generating the attractive force between the iron plate 32 and the magnet 15 and that is configured to close the flow path 14 by abutting against the opening portion 13 of the connection plug main body 10, wherein the magnet 15 is provided in such a manner that the magnitude of the magnetic force acting between the magnet 15 and the iron plate 32 is adjustable. As a result, since the attractive force pulling the lid portion 30 toward the connection plug main body 10 is variable, the force used to close the lid is adjustable.

The present invention has thus been explained on the basis of the embodiment; however, the present invention is not limited to this embodiment. For example, in the above embodiment, the magnet 15 is attached to the connection plug main body 10, while the iron plate 32 is attached to the lid portion 30; however, possible embodiments are not limited to this example. As long as attractive force is generated between the lid portion 30 and the connection plug main body 10, the combination of the magnetic members attached to the lid portion 30 and to the connection plug main body 10 may be different. An iron plate may be attached to the connection plug main body 10, while a magnet is attached to the lid portion 30. Alternatively, magnets may be attached to both the connection plug main body 10 and the lid portion 30.

Further, in the above embodiment, the magnetic force is adjusted by moving the magnet 15 of the connection plug main body 10 along the opening portion 13; however, possible embodiments are not limited to this example. As long as it is possible to adjust the magnetic force acting between the connection plug main body 10 and the lid portion 30, it is acceptable to adjust the magnetic force by moving an iron plate or a magnet attached to the lid portion 30 and thereby adjusting the distance from the opening portion 13.

Furthermore, in the above embodiment, the magnetic force is adjusted by moving the base portion 16 along the opening portion 13; however possible embodiments are not limited to this example. As long as it is possible to adjust the magnetic force acting between the connection plug main body 10 and the lid portion 30, it is also acceptable to adjust the magnetic force by configuring the magnetic members such as the magnet, the iron plate, and the like provided for the connection plug main body 10 and the lid portion 30 to be removable and replacing the magnet and/or the iron plate with a magnet having different magnetic force or with a magnet and/or an iron plate having a different size. Alternatively, it is also acceptable to adjust the magnetic force by changing the number of magnetic members, such as magnets or iron plates, attached to the connection plug main body 10 and to the lid portion 30.

### REFERENCE SIGNS LIST

- 10: connection plug main body
- 11: connection portion
- 12: base end portion
- 13: opening portion
- 14: flow path
- 15: magnet (main body-side magnetic member)
- 16: base portion
- 16A: annular portion
- 16B: circular cylindrical portion
- 17: anti-slip
- 18: lateral wall
- 19: clearance
- 19A: edge portion
- 30: lid portion
- 31: hinge portion
- 32: iron plate (lid-side magnetic member)
- 33: seal portion
- 100: medical instrument cap

## Claims

1. A medical instrument cap, comprising:
a connection plug main body that has formed therein a flow path penetrating from a base end portion formed to be connectable to an external member to an opening portion positioned on an opposite side and that has attached thereto a main body-side magnetic member; and
a lid portion that has attached thereto a lid-side magnetic member for generating attractive force between the lid-side magnetic member and the main body-side magnetic member and that is configured to close the flow path by abutting against the opening portion of the connection plug main body,
wherein at least one of the main body-side magnetic member and the lid-side magnetic member is configured so that magnitude of the attractive force acting between the main body-side magnetic member and the lid-side magnetic member is adjustable.

2. The medical instrument cap according to claim 1,
wherein the opening portion is formed to have a circular cylindrical shape and has a screw thread formed on an outer circumference thereof, and
wherein the main body-side magnetic member is attached to a base portion that is formed to have an annular shape and that has a screw thread formed on an inner circumference thereof, so as to be screwed together with the opening portion via the base portion.

3. The medical instrument cap according to claim 2,
Wherein the base portion includes a circular cylindrical portion to be screwed together with the opening portion.

4. The medical instrument cap according to claim 2,
wherein the base portion has an anti-slip formed along an outer circumference thereof.

5. The medical instrument cap according to claim 2,
wherein the connection plug main body has formed therein clearances through which an outer circumference of the base portion is exposed from a pair of locations positioned opposite to each other while being centered on an axial center of the opening portion.

6. The medical instrument cap according to claim 1,
wherein at least one of the main body-side magnetic member and the lid-side magnetic member is removably attached to the connection plug main body or the lid portion.
